# EUROPEAN PATENT APPLICATION

(11) **EP 3 150 187 A1**
(43) Date of publication of application: **05.04.2017**
(21) Application number: 16191932.9
(22) Date of filing: 30.09.2016
(51) Int. Cl.: A61K 8/34, A61K 8/36, A61Q 19/00

(54) **COMPOSITIONS AND METHODS FOR TREATING BLACKHEADS**

(30) Priority: 30.09.2015 US 201562234919 P
(71) Applicant: JOHNSON & JOHNSON CONSUMER INC., Skillman, NJ 08558 (US)
(72) Inventor: BATCHVAROVA, Nikoleta, Skillman, NJ New Jersey 08558 (US); JOHNSON, Diana, Skillman, NJ New Jersey 08558 (US); SHAH, Snehal M., Skillman, NJ New Jersey 08558 (US)
(74) Representative: Carpmaels & Ransford LLP

(57) **Abstract**

Leave-on compositions, articles of manufacture and methods of dissolving and otherwise treating blackheads are provided that employ neutralized fatty acids.

## Description

### FIELD OF THE INVENTION

The invention relates to leave-on compositions, articles of manufacture and methods for treating, in particular dissolving, blackheads using neutralized fatty acids.

### BACKGROUND OF THE INVENTION

Open comedones, or blackheads, are a primary sign of acne vulgaris, a disease of the hair follicles of the face, chest and back that affects a vast majority of humans during their lifetimes. A blackhead consists of a widened hair follicle filled with skin debris, bacteria and sebum and is capped with a blackened (oxidized) mass of skin debris. It is known that blackheads contain free fatty acids in particular. Nicolaides et al., Journal of Investigative Dermatology, Vol. 54, No. 6: 487-495 (1970).

Blackheads can be unsightly and cause embarrassment to the individual who suffers from them. In the past, blackhead sufferers have used skin cleansing products, including astringents, toners, surfactant-containing cleansers and the like, to cleanse and treat their skin daily. For example, NEUTROGENA Facial Cleansing Bar for Acne-Prone Skin is an acne rinse off product commercially available from Johnson & Johnson Consumer Inc. and contains triethanolamine, TEA stearate, sodium tallowate, glycerin, TEA-lauryl sulfate, sodium cocoate, water, sodium ricinoleate, sodium oleate, cetyl acetate, cocamide MEA, sodium stearate, BHT, tocopheryl acetate, tetrasodium etidronate, acetylated lanolin alcohol, trisodium HEDTA, and fragrance. PROACTIV Blackhead Dissolving Gel, commercially available from Guthy-Renker, contains salicylic acid (0.5%), water, dimethicone, cyclopentasiloxane, glycereth-26, glycerin, cyclohexasiloxane, PEG-80 sorbitan laurate, dimethiconol, polyacrylate-13, panthenol, camellia sinensis leaf extract, aloe barbadensis leaf juice, butyrospermum parkii (shea) butter, bisabolol, lactic acid, caprylyl glycol, ethylhexylglycerin, PEG-40 hydrogenated castor oil, polyisobutene, hexylene glycol, polysorbate 20, PPG-26-buteth-26, butyl methoxydibenzoylmethane, ethylhexyl methoxycinnamate, ethylhexyl salicylate, phenoxyethanol, and fragrance.

Exfoliating implements and compositions containing exfoliating ingredients to remove dead skin and improve the tone and texture of skin are also available on the market. For example, BIORE' Deep Cleansing Pore Strips, containing polyquaternium-37, silica, water, glycerin, polysilicone-13, PEG-12, dimethicone, titanium dioxide, *Hamamelis virginiana* (witch hazel) extract, butylene glycol, stearyl glycyrrhetinate, and methylparaben, are commercially available strips promoted to treat blackheads. However, these mechanical strips are not effective on every pore because they do not adhere homogeneously to the skin, and may also be painful to use.

The need remains for further effective blackhead treatments.

Applicants have now discovered that leave-on compositions containing a neutralized fatty acid are surprisingly effective at dissolving and otherwise treating blackheads. Accordingly, new methods, articles of manufacture and compositions for the treatment of blackheads are presented herein.

### SUMMARY OF THE INVENTION

The invention provides a method of dissolving a blackhead, comprising contacting the blackhead with a composition comprising a neutralized fatty acid.

The invention further provides a leave-on composition for treating blackheads, comprising a neutralized fatty acid and a topical carrier comprising a penetration enhancer.

The invention also provides an article of manufacture comprising: 1) a leave-on composition for treating blackheads comprising a neutralized fatty acid and a topical carrier comprising a penetration enhancer; and 2) a substrate.

The invention further provides a method of treating blackheads, comprising contacting skin in need of treatment for blackheads with a leave-on composition comprising a neutralized fatty acid and a topical carrier comprising a penetration enhancer.

The invention also provides a method of treating blackheads, comprising contacting skin in need of treatment for blackheads with an article of manufacture comprising: 1) a leave-on composition for treating blackheads comprising a neutralized fatty acid and a topical carrier comprising a penetration enhancer, and 2) a substrate.

### DETAILED DESCRIPTION OF THE INVENTION

The present methods, articles and compositions utilize leave-on formulations comprising one or more neutralized fatty acids for dissolving blackheads and treating skin in need of treatment for blackheads.

As used herein, "topically applying" means directly laying on or spreading on outer skin, e.g., by use of the hands or an applicator such as a wipe, puff, silicone scrublet or scrublet tip, roller, or spray. Such topical application may be applied to any skin in need of treatment for blackheads on the body, for example skin of the face, neck, chest, back, buttocks, arms, axilla, and/or legs, in particular the face, neck, chest and back.

As used herein, "blackhead" means any skin debris, bacteria and/or sebum, with or without a darkened (oxidized) mass at the tip, in a pore in skin, being either a clinical blackhead or a consumer-perceived blackhead.

As used herein, "skin in need of treatment for blackheads" means skin having blackheads or clogged pores.

As used herein, the term "treating" or "treatment" means the treatment (e.g., alleviation or elimination of symptoms and/or cure) and/or prevention or inhibition of a condition or disease.

As used herein, "essentially free" means containing 0.5% or less, more preferably about 0.1% or less, more preferably about 0.05 or less, more preferably about 0.01 % or less by weight. In one embodiment, a composition that is essentially free of an ingredient is totally free of the ingredient, *i.e.,* contains none of that ingredient.

As used herein, "cosmetically acceptable" means that the ingredients which the term describes are suitable for use in contact with tissues (e.g., the skin or hair) without undue toxicity, incompatibility, instability, irritation, allergic response, and the like.

The term "leave-on" as used herein means the compositions of this invention are intended to be applied to and remain on the skin. Leave-on compositions are to be distinguished from compositions applied to the skin and quickly removed either by washing, rinsing, wiping or the like, which are referred to as "rinse off compositions," as known in the art.

### Leave-On Compositions

The invention provides leave-on compositions for treating blackheads, comprising a neutralized fatty acid and a topical carrier comprising a penetration enhancer.

The neutralized fatty acid may be a saturated fatty acid. For example, the saturated fatty acid may have a carbon chain of C8 to C26. Examples include myristic acid, caprylic acid, capric, lauric acid, palmitic acid, stearic acid, arachidic acid, behenic acid, lignoceric acid, and cerotic acid.

The neutralized fatty acid may be an unsaturated fatty acid. For example, the unsaturated fatty acid may have a carbon chain of C14 to C22 with one or more double bonds. Examples include myristoleic acid, palmitoleic, sapienic acid, oleic acid, linoleic acid, alpha linoletic acid, arachidonic acid, eicosapentaenoic acid, eruicic acid, and docosahexaenoic acid.

The neutralized fatty acid may be a combination of neutralized saturated fatty acids and neutralized unsaturated fatty acids.

The neutralized fatty acid may be fully neutralized.

The neutralized fatty acid may be partially neutralized.

The neutralized fatty acid may comprise a combination of fully neutralized and partially neutralized fatty acids.

In one embodiment, the neutralized fatty acid has been neutralized with a neutralizing agent selected from the group consisting of sodium hydroxide, potassium hydroxide, calcium hydroxide, triethanolamine, trolamine, aminomethylpropanol, sodium bicarbonate, and the like.

In another embodiment, the neutralized fatty acid has been neutralized with a neutralizing agent at a mole ratio of fatty acid to neutralizing agent of about 1:0.75 to about 1:1.25.

The composition generally may comprise about 2 to about 15 % by weight of the neutralized fatty acid based on the total weight of the composition. In particular, the composition may comprise about 5% to about 10% by weight of the neutralized fatty acid or fatty acid combinations.

The penetration enhancer increases skin permeability by reversibly damaging or by altering the physiochemical nature of the stratum corneum to reduce its diffusional resistance. The penetration enhancer alters the physical or chemical nature of the stratum corneum (top layer of skin) to allow the neutralized fatty acid to penetrate this layer to reach the lower layers of skin.

Examples of penetration enhancers include, but are not limited to, propylene glycol, ethanol, essential oils such as linalool and eugenol, alkyl lactates, cetyl lactate and dimethyl isosorbide.

The composition generally may comprise about 0.1 to about 50 % by weight of the penetration enhancer based on the total weight of the composition. In particular, the composition may comprise about 0.5 to about 10 % by weight of the penetration enhancer based on the total weight of the composition.

The leave-on composition may further comprise any of a variety of additional cosmetically active agents used in leave-on compositions, as known in the art.

In one embodiment, the composition further comprises an encapsulation agent. The encapsulation agent is capable of encapsulating the composition, including any additional active agents such as anti-acne agents, for example salicylic acid.

In another embodiment, the composition further comprises a lipid dissolver.

Examples of suitable additional active agents include: skin lightening agents, darkening agents, anti-aging agents, tropoelastin promoters, collagen promoters, anti-acne agents, anti-rosacea agents, shine control agents, antimicrobial agents such as anti-yeast agents, anti-fungal, and anti-bacterial agents, anti-inflammatory agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth enhancing agents, hair growth delaying agents, firming agents, hydration boosters, efficacy boosters, anti-callous agents, agents for skin conditioning, anti-cellulite agents, odor-control agents such as odor masking or pH changing agents, and the like.

Examples of various suitable additional cosmetically acceptable actives include hydroxy acids; benzoyl peroxide; D-panthenol; UV filters such as but not limited to avobenzone (Parsol 1789), bisdisulizole disodium (Neo Heliopan AP), diethylamino hydroxybenzoyl hexyl benzoate (Uvinul A Plus), ecamsule (Mexoryl SX), methyl anthranilate, 4-aminobenzoic acid (PABA), cinoxate, ethylhexyl triazone (Uvinul T 150), homosalate, 4-methylbenzylidene camphor (Parsol 5000), octyl methoxycinnamate (Octinoxate), octyl salicylate (Octisalate), padimate O (Escalol 507), phenylbenzimidazole sulfonic acid (Ensulizole), polysilicone-15 (Parsol SLX), trolamine salicylate, Bemotrizinol (Tinosorb S), benzophenones 1-12, dioxybenzone, drometrizole trisiloxane (Mexoryl XL), iscotrizinol (Uvasorb HEB), octocrylene, oxybenzone (Eusolex 4360), sulisobenzone, bisoctrizole (Tinosorb M), titanium dioxide, zinc oxide; carotenoids; free radical scavengers; spin traps; retinoids and retinoid precursors such as 30 retinol, retinoic acid and retinyl palmitate; ceramides; polyunsaturated fatty acids; essential fatty acids; enzymes; enzyme inhibitors; minerals; hormones such as estrogens; steroids such as hydrocortisone; 2-dimethylaminoethanol; copper salts such as copper chloride; peptides containing copper such as Cu:Gly-His-Lys, coenzyme Q10; amino acids such a proline; vitamins; lactobionic acid; acetyl-coenzyme A; niacin; riboflavin; thiamin; ribose; electron transporters such as NADH and FADH2; and other botanical extracts such as oat, aloe vera, Feverfew, Soy, Shiitake mushroom extracts, and derivatives and mixtures thereof.

Examples of anti-acne and anti-rosacea agents include, but are not limited to: retinoids such as tretinoin, isotretinoin, motretinide, adapalene, tazarotene, azelaic acid, and retinol; salicylic acid; benzoyl peroxide; resorcinol; sulfur; sulfacetamide; urea; antibiotics such as tetracycline, clindamycin, metronidazole, and erythromycin; anti-inflammatory agents such as corticosteroids (e.g., hydrocortisone), ibuprofen, naproxen, and hetprofen; and imidazoles such as ketoconazole and elubiol; and salts and prodrugs thereof. Other examples of anti-acne active agents include essential oils, alpha-bisabolol, dipotassium glycyrrhizinate, camphor, β-glucan, allantoin, feverfew, flavonoids such as soy isoflavones, saw palmetto, chelating agents such as EDTA, lipase inhibitors such as silver and copper ions, hydrolyzed vegetable proteins, inorganic ions of chloride, iodide, fluoride, and their nonionic derivatives chlorine, iodine, fluorine, and other valences, synthetic phospholipids and natural phospholipids such as Arlasilk™ phospholipids CDM, SV, EFA, PLN, and GLA (Uniqema, ICI Group of Companies, Wilton, UK), and combinations of two or more thereof.

Examples of suitable skin lightening active agents include, but are not limited to, tyrosinase inhibitors, melanin-degradation agents, melanosome transfer inhibiting agents including PAR-2 antagonists, exfoliants, sunscreens, retinoids, antioxidants, Tranexamic acid, tranexamic acid cetyl ester hydrochloride, skin bleaching agents, linoleic acid, adenosine monophosphate disodium salt, Chamomilla extract, allantoin, opacifiers, talcs and silicas, zinc salts, and the like, and other agents as described in Solano et al. Pigment Cell Res. 19 (550-571) and Ando et al. Int J Mol Sci 11 (2566-2575).

Examples of suitable tyrosinase inhibitors include but, are not limited to, Vitamin C and its derivatives, Vitamin E and its derivatives, Kojic Acid, Arbutin, resorcinols, hydroquinone, Flavones e.g. Licorice flavanoids, Licorice root extract, Mulberry root extract, Dioscorea Coposita root extract, Saxifraga extract and the like, Ellagic acid, Salicylates and derivatives, Glucosamine and derivatives, Fullerene, Hinokitiol, Dioic acid, Acetyl glucosamine, 5,5'-dipropyl-biphenyl-2,2'-diol (Magnolignan), 4-(4-hydroxyphenyl)-2-butanol (4-HPB), combinations of two or more thereof, and the like. Examples of vitamin C derivatives include, but are not limited to, ascorbic acid and salts, Ascorbic Acid-2-Glucoside, sodium ascorbyl phosphate, magnesium ascorbyl phosphate, and natural extract enriched in vitamin C. Examples of vitamin E derivatives include, but are not limited to, alpha-tocopherol, beta, tocopherol, gamma-tocopherol, delta-tocopherol, alpha-tocotrienol, beta-tocotrienol, gamma-tocotrienol, delta-tocotrienol and mixtures thereof, tocopherol acetate, tocopherol phosphate and natural extracts enriched in vitamin E derivatives. Examples of resorcinol derivatives include, but are not limited to, resorcinol, 4-substituted resorcinols like 4-alkylresorcinols such as 4-butyresorcinol (rucinol), 4-hexylresorcinol (Synovea HR, Sytheon), phenylethyl resorcinol (Symwhite, Symrise), 1-(2,4-dihydroxyphenyl)-3-(2,4-dimethoxy-3-methylphenyl)-Propane (Nivitol, Unigen) and the like and natural extracts enriched in resorcinols. Examples of salicylates include, but are not limited to, 4-methoxy potassium salicylate, salicylic acid, acetylsalicylic acid, 4-methoxysalicylic acid and their salts. In certain preferred embodiments, the tyrosinase inhibitors include a 4-substituted resorcinol, a vitamin C derivative, or a vitamin E derivative. In more preferred embodiments, the tyrosinase inhibitor comprises Phenylethyl resorcinol, 4-hexyl resorcinol, or ascorbyl-2-glucoside.

Examples of suitable melanin-degradation agents include, but are not limited to, peroxides and enzymes such as peroxidases and ligninases. In certain preferred embodiments, the melanin-inhibiting agents include a peroxide or a ligninase.

Examples of suitable melanosome transfer inhibiting agents including PAR-2 antagonists such as soy trypsin inhibitor or Bowman-Birk Inhibitor, Vitamin B3 and derivatives such as Niacinamide, Essential soy, Whole Soy, and Soy extract. In certain preferred embodiments, the melanosome transfer inhibiting agents includes a soy extract or niacinamide.

Examples of exfoliants include, but are not limited to, alpha-hydroxy acids such as lactic acid, glycolic acid, malic acid, tartaric acid, citric acid, or any combination of any of the foregoing, beta-hydroxy acids such as salicylic acid, polyhydroxy acids such as lactobionic acid and gluconic acid, and mechanical exfoliation such as microdermabrasion. In certain preferred embodiments, the exfoliants include glycolic acid or salicylic acid.

Examples of sunscreens include, but are not limited to, avobenzone (Parsol 1789), bisdisulizole disodium (Neo Heliopan AP), diethylamino hydroxybenzoyl hexyl benzoate (Uvinul A Plus), ecamsule (Mexoryl SX), methyl anthranilate, 4-aminobenzoic acid (PABA), cinoxate, ethylhexyl triazone (Uvinul T 150), homosalate, 4-methylbenzylidene camphor (Parsol 5000), octyl methoxycinnamate (Octinoxate), octyl salicylate (Octisalate), padimate O (Escalol 507), phenylbenzimidazole sulfonic acid (Ensulizole), polysilicone-15 (Parsol SLX), trolamine salicylate, Bemotrizinol (Tinosorb S), benzophenones 1-12, dioxybenzone, drometrizole trisiloxane (Mexoryl XL), iscotrizinol

(Uvasorb HEB), octocrylene, oxybenzone (Eusolex 4360), sulisobenzone, bisoctrizole (Tinosorb M), titanium dioxide, zinc oxide, and the like.

Examples of retinoids include, but are not limited to, retinol (Vitamin A alcohol), retinal (Vitamin A aldehyde), retinyl acetate, retinyl propionate, retinyl linoleate, retinoic acid, retinyl palmitate, isotretinoin, tazarotene, bexarotene, Adapalene, combinations of two or more thereof and the like. In certain preferred embodiments, the retinoid is selected from the group consisting of retinol, retinal, retinyl acetate, retinyl propionate, retinyl linoleate, and combinations of two or more thereof. In certain more preferred embodiments, the retinoid is retinol.

Examples of antioxidants include, but are not limited to, water-soluble antioxidants such as sulfhydryl compounds and their derivatives (e.g., sodium metabisulfite and N-acetyl cysteine, glutathione), lipoic acid and dihydrolipoic acid, stilbenoids such as resveratrol and derivatives, lactoferrin, iron and copper chelators and ascorbic acid and ascorbic acid derivatives (e.g., ascobyl-2-glucoside, ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinones. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, black tea, white tea, pine bark, feverfew, parthenolide-free feverfew, oat extracts, blackberry extract, cotinus extract, soy extract, pomelo extract, wheat germ extract, Hesperedin, Grape extract, Portulaca extract, Licochalcone, chalcone, 2,2'-dihydroxy chalcone, Primula extract, propolis, and the like.

Examples of suitable anti-inflammatory agents include substituted resorcinols, (E)-3-(4-methylphenylsulfonyl)-2-propenenitrile (such as "Bay 11-7082," commercially available from Sigma-Aldrich of St. Louis, Missouri), tetrahydrocurcuminoids (such as Tetrahydrocurcuminoid CG, available from Sabinsa Corporation of Piscataway, NJ), extracts and materials derived from the following: Phellodendron amurense Cortex Extract (PCE), Non-Denatured Soy (Glycine max), Feverfew (Tanacetum parthenium), Ginger (Zingiber officinale), Ginko (Ginkgo biloba), Madecassoside (Centella asiatica extract ingredient), Cotinus (Cotinus coggygria), Butterbur Extract (Petasites hybridus), Goji Berry (Lycium barbarum), Milk Thistle Extract (Silybum marianum), Honeysuckle (Lonicera japonica), Basalm of Peru (Myroxylon pereirae), Sage (Salvia officinalis), Cranberry Extract (Vaccinium oxycoccos), Amaranth Oil (Amaranthus cruentus), Pomegranate (Punica granatum), Yerbe Mate (Ilex paraguariensis Leaf Extract), White Lily Flower Extract (Lilium candidum), Olive Leaf Extract (Olea europaea), Phloretin (apple extract), Oat Flour (Aveena sativa), Lifenol (Hops: Humulus lupulus) Extract, Bugrane P (Ononis spinosa), Licochalcone (Licorice: Glycyrrhiza inflate extract ingredient), Symrelief (Bisabolol and Ginger extract), combinations of two or more thereof, and the like.

In one embodiment, the anti-inflammatory agent is a resorcinol. Particularly suitable substituted resorcinols include 4-hexyl resorcinol and 4-octylresorcinol, particularly 4-hexyl resorcinol. 4-Hexyl resorcinol is commercially available as "SYNOVEA HR" from Sytheon of Lincoln Park, NJ. 4-Octylresorcinol is commercially available from City Chemical LLC of West Haven, Connecticut.

By "extracts of feverfew," it is meant extracts of the plant "Tanacetum parthenium," such as may be produced according to the details set for the in US Patent Application Publication No. 2007/0196523, entitled "PARTHENOLIDE FREE BIOACTIVE INGREDIENTS FROM FEVERFEW (TANACETUM PARTHENIUM) AND PROCESSES FOR THEIR PRODUCTION." One particularly suitable feverfew extract is commercially available as about 20% active feverfew, from Integrated Botanical Technologies of Ossining, NY.

What is meant by an emollient is a compound that helps to maintain the soft, smooth, and pliable appearance of the skin (e.g., by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY), and include, but are not limited to, petrolatum, hexyldecyl stearate and plant, nut, and vegetable oils such as macadamia nut oil, rice bran oil, grape seed oil, palm oil, prim rose oil, hydrogenates peanut oil, and avocado oil.

What is meant by a humectant is a compound intended to increase the water content of the top layers of skin (e.g., hygroscopic compounds). Examples of suitable humectants include those found Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY) and include, but are not limited to, glycerin, sorbitol or trehalose (e.g., α,α- trehalose, β,β-trehalose, α,β-trehalose) or a salt or ester thereof (e.g., trehalose 6-phosphate).

What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc., New York, NY) and include, but are not limited to anionic surfactants such as sulfates, cationic surfactants such as betaines, amphoteric surfactants such as sodium coco glycinate, noionic surfactants such as alkyl polyglucosides.

Examples of suitable chelating agents include those which are capable of protecting and preserving the compositions of this invention. Preferably, the chelating agent is ethylenediamine tetracetic acid ("EDTA"), and more preferably is tetrasodium EDTA, available commercially from Dow Chemical Company of Midland, Michigan under the trade name, "Versene 100XL."

Suitable preservatives include, for example, parabens, quaternary ammonium species, phenoxyethanol, benzoates, DMDM hydantoin, organic acids and are present in the composition in an amount, based upon the total weight of the composition, from about 0 to about 1 percent or from about 0.05 percent to about 0.5 percent.

Any of a variety of conditioners which impart additional attributes, may be suitable for use in this invention. Examples include, but are not limited to, volatile silicone conditioning agent having an atmospheric pressure boiling point less than about 220°C. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof, and preferably include cyclomethicone fluids. Other suitable conditioners include cationic polymers, including polyquarterniums, cationic guar, and the like.

Any of a variety of commercially available pearlescent or opacifying agents may be suitable for use in the composition. Examples of suitable pearlescent or opacifying agents include, but are not limited to, mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)a-H, wherein J is an alkylene group having from about 2 to about 3 carbon atoms; and a is 2 or 3; fatty alcohols containing from about 16 to about 22 carbon atoms; fatty esters of the formula: KCOOCH₂L, wherein K and L independently contain from about 15 to about 21 carbon atoms; inorganic solids insoluble in the shampoo composition, and mixtures thereof.

Any fragrances suitable for use on skin may be used in accordance with the present invention.

The additional cosmetically active agent may be present in the composition in any suitable amount, for example, in an amount of from about 0.0001 % to about 20% by weight of the composition, e.g., about 0.001 % to about 10% such as about 0.01% to about 5%. In certain preferred embodiments, in an amount of 0.1% to 5% and in other preferred embodiments from 1% to 2%.

In one embodiment, the composition of the present invention comprises a high melting point lactate. Examples of such lactates include, but are not limited to, long carbon chain lactates such as C16-C35 lactates, such as cetyl lactate. The amount of such lactates in the composition of the present invention may vary from about 0.1 % to about 50%, for example from about 0.5% to about 20%, or from about 1% to about 10% by weight, based on the total weight of the composition.

In one embodiment, the composition includes a sebum miscible agent. As used herein, a "sebum miscible agent" is an agent that is miscible with sebum as determined by the following assay. Artificial sebum is prepared as set forth on page 79 (Table 5.4) of a book chapter entitled "The Influence of Skin Surface Lipids on Topical Formulations" by Obsorne and Hatzenbuhler (in "Topical Drug Delivery Formulations", edited by D. Osborne and A. Amann, Marcel Dekker, Inc., New York, 1990, pages 69-85). At room temperature this sebum is a white waxy substance. 50 .mu.l of the sebum is deposited into a 200 .mu.l clear vial using a precision micropipette. 100 .mu.l of the test agent is then added to the vial. The vial is warmed at 32° C and visually inspected at baseline and at eight hours. If the agent is miscible with the sebum, the sebum will become transparent.

The following are non-limiting examples of sebum miscible agents: aromatic alcohols such as phenyl alcohols with chemical structures of C₆H₅-R(OH) where R is an aliphatic radical, such as benzyl alcohol and phenethyl alcohol; aromatic glycol ethers such as ethylene glycol phenyl ether; propylene or butylene oxide-based glycol ethers such as propylene glycol methyl ether and those disclosed in U.S. Pat. No. 5,133,967; fatty acids, polyunsaturated fatty acids such as linoleic acid, linolenic acid, stearidonic acid, plant, fruit, or marine derived extracts rich in essential fatty acid or polyunsaturated fatty acids such as but not limited to vaccinium myrtillus (bilberry) seed oil, vaccinium macrocarpon (cranberry) seed oil, vaccinium vitis-idaea (lingonberry) seed oil, rubus idaeus (raspberry) seed oil, rubus chamaemorus (cloudberry) seed oil, ribes nigrum (black currant) seed oil, hippophae rhamnoides (sea buckthorn) seed oil, echium plantagineum (echium) seed oil, hordeum vulgare (barley) seed oil, betula alba bud extract, saw palmetto extract, borage oil, evening primrose oil, witch hazel extract and soy oil; cetyl ocenate; isostearyl benzoate; pentaerythiol teraoctenate; isostearyl benzoate; methyl gluceth; tocopherol acetate; benzalkonium chloride; and benzethonium chloride, and combinations thereof.

In one embodiment, the compositions of the present invention include an antimicrobial agent. What is meant by an antimicrobial agent is a compound that kills microorganisms or prevents or inhibits their growth or reproduction. Examples of antimicrobial agents include, but are not limited to: ethanol, propanol, betains, benzalkonium chloride, benzethonium chloride, lauric arginayte, sugarquat, methyl benzethonium chloride, cetypyridiunium chloride, 2,4,4',-trichloro-2-hydroxy diphenyl ether (Triclosan), parachlorometa xylenol (PCMX), lodopropynyl butylcarbamate, diazolidinyl urea, chlorhexidene digluconate, chlorhexidene acetate, chlorhexidene isethionate, chlorhexidene hydrochloride, hexetidine, Quaternium 15, triclocarbon, polyhexamethylene biguanide, cetylpyridium chloride, imidazolidinyl urea, diazolidinyl urea, 3-iodo-2-propynyl-N-butylcarbamate, 2-methyl-4-isothiazolin-3-one, dimethyl dimethyl hydantoin,(5-chloro-2-(2,4-dichlorophenoxy)phenol, monolaurin glyceryl laurate, camellia sinensis, candida bombicola/glucose/methyl rapeseedate ferment, hydrogen peroxide, phenol, poloxamer 188, PVP-iodine, thiourea, natural antimicrobial agents, such as cinnamon oil, cinnamaldehyde, lemongrass oil, clove oil, saw palmetto extract, thyme oil white, thyme oil red, thymol, tea tree oil, pinus pinaster bark extract, rosemary leaf extract, grape seed extract, and betel oil, silver containing compounds, such as silver nitrate, silver lactate, silver citrate, and silver zeolite, antimicrobial fatty acid ester of a polyhydric alcohol, a fatty ether of a polyhydric alcohol and alkoxylated derivatives thereof, and combinations thereof.

In one embodiment, the amount of antimicrobial agent in the composition is from about 0.001% to about 10%, such as from about 0.01% to about 5% such as from about 0.05% to about 2% by weight, based on the total weight of the composition.

In one embodiment the antimicrobial agent is an anti-fungal agent such as an azole. Examples include, but are not limited to, miconazole, ketoconazole, econazole, itraconazole, sertaconazole, fluconazole, voriconazole, clioquinol, bifoconazole, terconazole, butoconazole, tioconazole, oxiconazole, sulconazole, saperconazole, clotrimazole, undecylenic acid, haloprogin, butenafine, tolnaftate, nystatin, ciclopirox olamine, terbinafine, amorolfine, naftifine, elubiol, griseofulvin, their cosmetically acceptable salts, and combinations thereof.

In one embodiment the antimicrobial agent is an antibiotic or an antiseptic. Examples include, but are not limited to, mupirocin, neomycin sulfate bacitracin, polymyxin B, 1-ofloxacin, tetracyclines such as chlortetracycline hydrochloride, oxytetracycline-10 hydrochloride and tetrachcycline hydrochoride, clindamycin phosphate, gentamicin sulfate, metronidazole, hexylresorcinol, methylbenzethonium chloride, phenol, quaternary ammonium compounds, tea tree oil, and combinations thereof.

In one embodiment, the compositions of the present invention include an antipsoriatic agent. Examples of antipsoriatic agents include, but are not limited to, corticosteroids (e.g., betamethasone dipropionate, betamethasone valerate, clobetasol propionate, diflorasone diacetate, halobetasol propionate, triamcinonide, dexamethasone, fluocinonide, fluocinolone acetonide, halcinonide, triamcinolone acetate, hydrocortisone, hydrocortisone venerate, hydrocortisone butyrate, aclometasone dipropionte, flurandrenolide, mometasone furoate, and methylprednisolone acetate), methotrexate, cyclosporine, calcipotriene, anthraline, shale oil, elubiol, ketoconazole, coal tar, salicylic acid, zinc pyrithione, selenium sulfide, hydrocortisone, sulfur, , 2,2'-sulfanediylbis(4,6-dichlorophenol) (bithionol), 6-hydroxy-1,3-benzoxathiol-2-one (tioxolone), 2,7-dimethylthianthrene (mesulfen), menthol, and pramoxine hydrochloride, and combinations thereof.

In one embodiment, the compositions of the present invention include an anti-viral agent. Examples of anti-viral agents include, but are not limited to, imiquimod, podofilox, podophyllin, interferon alpha, acyclovir, famcyclovir, valcyclovir, reticulos and cidofovir.

In one embodiment, the composition of the present invention includes an anti- dandruff agent. Examples of anti-dandruff agents include but are not limited to zinc pyrithione, elubiol, coal tar, salicylic acid or selenium sulfide, sulfur, ketoconazole, corticosteroids such as fluocinolone acetonide, caffeine and combinations thereof.

In one embodiment, the composition of the present invention includes active agents for treating keratosis pilaris. Examples of active agents for treating keratosis pilaris include but are not limited to fluoracil, Imiquimod, aminolevulinic acid and combinations thereof.

In one embodiment, the composition of the present invention includes hair growth regulating agents for helping or retarding hair growth, to treat alopecia and hirsutism, such as a 5-alpha reductase inhibitor. Examples of 5-alpha reductase inhibitors include but are not limited to finasteride, dutasteride, ketoconazole, minoxidil and natural extracts such as but not limited to soy and isoflavones.

The compositions of the present invention may further include an alcohol. Examples of suitable alcohols include, but are not limited to, ethyl alcohol. In one embodiment, the composition includes less than 40%, such as from about 0.01% to about 40%, for example from about 0.1 % to about 30%, or from about 1 % to about 20% by weight, of alcohol based on the total weight of the composition.

In another embodiment, the composition may include compounds that improve aesthetics, for example the feel, texture, or smoothness of the composition. Examples of suitable compounds include but are not limited to silica, hydrated silica, colloidal silica, aluminum sulfate, and combinations thereof.

In another embodiment, the composition may include comprise cooling or warming agents. Examples of cooling or warming agents include but are not limited to menthol, zeolites and combinations thereof.

The compositions may be made into a wide variety of leave on-product types that include but are not limited to solid and liquid compositions such as lotions, creams, gels, sticks, sprays, shaving creams, ointments, pastes, powders, mousses, masks, peels, make-ups, and wipes. These product types may comprise any one of several types of cosmetically acceptable topical carriers including, but not limited to solutions, emulsions (e.g., microemulsions and nanoemulsions), gels, solids and liposomes.

The compositions may be used in conjunction with devices such as skin abrading, skin messaging, electro-stimulation devices, light-therapy devices, ultrasound devices, radio frequency devices, thermal/cooling devices, and micro-penetration devices.

The following are examples of topical carriers that may be used according to the invention. Other carriers can be formulated by those of ordinary skill in the art.

The compositions useful in the present invention can be formulated as solutions. Solutions typically include an aqueous solvent (e.g., from about 50% to about 99% or from about 90% to about 95% of a cosmetically acceptable aqueous solvent).

Such solutions typically contain an emollient, for example about 2% to about 50% by weight of emollient(s). As used herein, "emollients" refer to materials used for the prevention or relief of dryness, as well as for the protection of the skin. A wide variety of suitable emollients are known and may be used herein. See CTFA Handbook which contains numerous examples of suitable materials.

The compositions may be formulated as lotions, comprising a humectant such as propylene glycol, butylene glycol, hexylene glycol, glycerin, or those listed in CTFA handbook. Lotions typically comprise from about 1% to about 20% (e.g., from about 5% to about 10%) of an emollient(s) and from about 50% to about 90% (e.g., from about 60% to about 80%) of water.

The compositions may be formulated as creams. A cream typically comprises from about 5% to about 50% (e.g., from about 10% to about 20%) of an emollient(s) and from about 45% to about 85% (e.g., from about 50% to about 75%) of water.

The compositions may be formulated as ointments. An ointment may comprise a simple base of animal or vegetable oils or semi-solid hydrocarbons. An ointment may comprise from about 2% to about 10% of an emollient(s) plus from about 0.1 % to about 2% of a thickening agent(s). A more complete disclosure of thickening agents or viscosity increasing agents useful herein can be found in the CTFA Handbook pp. 1693-1697.

The compositions may be formulated as emulsions. If the topical carrier is an emulsion, from about 1% to about 10% (e.g., from about 2% to about 5%) of the topical carrier comprises an emulsifier(s). Emulsifiers may be nonionic, anionic or cationic. Suitable emulsifiers are disclosed in, for example, CTFA Handbook, pp. 1673-1686.

Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s). Single emulsion skin care preparations, such as lotions and creams, of the oil-in-water type and water-in-oil type are well-known in the cosmetic art and are useful as topical carriers for the invention. Multiphase emulsion compositions, such as the water-in-oil-in-water type are also useful in the subject invention. In general, such single or multiphase emulsions contain water, emollients, and emulsifiers as essential ingredients.

The compositions can also be formulated as gels (e.g., an aqueous gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprise between about 0.1% and 5%, by weight, of such gelling agents.

In one embodiment, the composition is anhydrous. In one embodiment, such anhydrous composition is exothermic upon application.

The compositions of the present invention can also be formulated into a solid formulation (e.g., a wax-based stick, powder, a wipe containing powder, or a dressing).

The compositions may also contain, in addition to the above components, a wide variety of additional oil-soluble materials and/or water-soluble materials conventionally used in compositions for use on the skin at their art-established levels.

### Films and Other Articles of Manufacture

In one embodiment the invention provides an article of manufacture comprising a leave-on composition for treating blackheads comprising a neutralized fatty acid and a substrate.

The substrate may take any one of a variety of forms. For example, the substrate may be a solid, semi-solid, or dissolvable substrate. It may be for instance a film, wipe, mask, pad, glove, strip, patch, and may have any shape as desired.

The substrate may be made from any one of a variety of materials, such as wovens, nonwovens, polymers, and silicones as known in the art.

Preferably, the substrate is comfortable, flexible, easy to apply to the blackhead or skin in need of treatment for blackheads, and capable of remaining in place for an extended period of time.

In one embodiment, the substrate is a wipe, glove, or a facial mask. Preferably, such embodiments comprise a water-insoluble substrate, for example a nonwoven. For certain embodiments, the water-insoluble substrate may have a size and shape such that it covers the face of a human user to facilitate placing the water-insoluble substrate about the face of the user as a mask substrate. For example, the water-insoluble mask substrate may have openings for a mouth, nose, and/or eyes of the user. Alternatively, the water insoluble substrate may have no such openings. Such a configuration without openings may be useful for embodiments of the invention in which the water-insoluble substrate is intended to be draped over a non-facial expanse of skin or if the water-insoluble substrate is intended to be used as wipe. The water-insoluble substrate may have various shapes, such as an angular shape (e.g., rectangular) or an arcuate shape such as circular or oval. For certain embodiments, the substrate is a glove such as described in U.S. Published Application No 2006/0141014 which is incorporated herein in its entirety. In one embodiment of the invention, the product includes a plurality of water-insoluble substrates of different shapes.

In one embodiment, the substrate comprises a film. As used herein, the term "film" means a composition that forms a thin layer or membrane on mammalian and, more particularly, human skin. Such film may comprise a single layer or multiple layers.

In one embodiment the substrate comprises a dissolvable film. A variety of dissolvable films are known in the art, and any one of these may be used according to the invention.

In one particular embodiment, the article of manufacture may be an integral film product as described in US 2015/0182991, the disclosure of which is incorporated herein by reference. The integral film product is arranged and configured to be removable from the manufacturing substrate it is made on, for use independent of the manufacturing substrate. In particular, the product may be made by placing a mask over a manufacturing substrate having a releasable surface, delivering a film-forming composition through the mask to form a raw shape on the manufacturing substrate; removing the mask; and solidifying the raw shape into the integral film product disposed on the manufacturing substrate. The mask has at least one aperture having a shape corresponding to the desired integral film product. The integral film product is arranged and configured to be removable from the releasable surface of the manufacturing substrate for use independent thereof.

In another embodiment, the article of manufacture may be a multilayered shaped film product as described in US 2015/0182990, the disclosure of which is incorporated herein by reference. For example, the article of manufacture may comprise a two layer shaped film product comprising a first surface comprising the neutralized fatty acid as a benefit agent to be delivered to a blackhead or skin in need of treatment for blackheads, and a second surface exposed to the exterior. The article of manufacture may be made using a process that comprises delivering liquid film-forming compositions through a mask; removing the mask to leave a multilayered raw shape; and curing the multilayered raw shape to form the multilayered shaped film product. The mask has a delivery surface, an opposite surface and at least one aperture having a design corresponding to the desired shaped film product. The film-forming compositions are delivered through a multistream nozzle. The movement of the mask and the delivery of the first and second liquid film-forming compositions to the mask aperture are controlled to provide a volumetric flow rate of the first and second liquid film-forming compositions to the mask aperture corresponding to the volume of a void. The nozzle is in contact with the delivery surface of the mask.

In another particular embodiment, the article of manufacture may be a multilayered film product as described in US 2015/0182992, the disclosure of which is incorporated herein by reference. For example, the article of manufacture may comprise a two layer shaped film product in which a first layer has a larger surface area than a second layer disposed on the first layer. This forms an "island" of the second layer on top of the first layer. One of the two layers is for contacting a blackhead or skin in need of treatment for blackheads and comprises the neutralized fatty acid as a benefit agent. The other layer is exposed to the exterior. The article of manufacture may be made by a process that comprises delivering a first film-forming composition through a first mask to form a first raw shape; removing the first mask; placing a second mask over the first raw shape; delivering a second film-forming composition through the second mask to form a second raw shape on the first raw shape; removing the second mask; and solidifying the first and second raw shapes to provide the shaped film product.

In a further embodiment, the article of manufacture may be a shaped film product as described in US 2015/0182993, the disclosure of which is incorporated herein by reference. Such shaped film product may be made by placing a mask over a manufacturing substrate; delivering a film-forming composition through a nozzle to form a raw shape on the manufacturing substrate; removing the mask; and solidifying the film-forming composition to provide the shaped film product disposed on the manufacturing substrate. The mask has a delivery surface and an opposite manufacturing substrate-facing surface and at least one aperture having a design corresponding to the desired shaped film product. The nozzle is disposed in sealing engagement with the delivery surface of the mask to the at least one aperture of the mask during delivery of the film-forming composition.

In yet another embodiment, the article of manufacture comprises a multilayer topically applied film as described in US Provisional Application No. 62/182,888, the disclosure of which is incorporated herein by reference. This film is readily removable upon application of water thereto. As used herein, "readily removable" means the article may dissolve or disintegrate upon application of water to the article, such that it may be removed from the skin without scrubbing or the like.

The article comprises a first top layer having a top surface for facing outwardly from the skin and a second bottom surface opposite the top surface for facing towards the skin. The article also comprises a second bottom skin-contacting layer comprising a first surface for facing and adhered to the bottom surface of the first top layer and a second outwardly-facing surface for contacting and adherence of the article to the skin when the article is applied thereto. The bottom skin-contacting layer comprises the neutralized fatty acid. In addition, each of the first top layer and second bottom layer comprises a water-soluble film former and the article is readily removable from the skin upon application of water thereto.

This article of manufacture may be formed by one of the above-described processes of forming multilayer shaped film products. It may also be made by casting and drying an adhesive layer, and then casting the top layer on top of the bottom layer. The two layers may adhere to one another by any of the known methods of adhesion (mechanical, chemical, dispersive, electrostatic, diffusive, etc.). In one embodiment, the two layers preferably are both water soluble, so that the water in the non-adhesive outwardly-facing layer will slightly dissolve the already dried adhesive skin contacting layer, thereby creating a certain amount of diffusive adhesion at the interface of the two layers. In a second embodiment, both layers are cast wet on wet, and intermixing of the materials occurs at their interface, thereby creating a bond by diffusive adhesion. Preferably, the materials have a common solvent and / or are miscible with each other so that they intermix and bond together. It will be appreciated that the materials of the adhesive and non-adhesive layers (the skin-contacting and outwardly-facing layers, respectively) may have a common solvent other than water, such as alcohol, so that the materials bond to each other.

For example, the skin-contacting layer preferably comprises a hydrophilic film-forming polymer, a solubilizing agent to solubilize other ingredients in the film, a disintegration promoter, a thickening agent/ structuring agent/ texture modifier, a hydroscopic agent/wetting agent to retain skin moisture, a partition coefficient modifier/ absorption-or permeation-promoting substances to drive the hydroscopic agent into skin, a plasticizer/primary adhesive agent for flexibility and softness, a solvent used for hydrocolloids and retain latent moisture and keep final article flexible and other auxiliaries or additives. The skin-contacting layer is applied preferably directly to the skin surface and possesses properties suitable for use as the skin-contacting surface of the article. Such properties include rapid dissolution, sustained adhesion strength, semi-occlusiveness, and flexibility. The skin-contacting layer comprises the neutralized fatty acid and may further comprise cosmetically active agents which can be delivered to the skin by the skin contacting layer.

The outwardly-facing layer possesses proprieties suitable for use as a physical barrier for the multi-layer topical skin article, allowing it to remain clean of dust and dirt and debris while the article remains in place on the application site. Such proprieties include rapid dissolution, semi-occlusiveness, flexibility, and non-stickiness. The outwardly-facing layer comprises a hydrophilic film forming polymer, a disintegration promoter, an oil-in-water emulsifier, a wax to limit water migration from the skin contacting layer to the topical layer, a plasticizer for flexibility and softness, a primary adhesive agent, a solvent used for hydrocolloids and to retain latent moisture and to keep the final article flexible, and other auxiliaries or additives.

In another embodiment, article of manufacture comprises a non-dissolving, peelable film as described in US 2014/0093587, the disclosure of which is incorporated herein by reference. As used herein, the term "peelable" means removably applied and adhered to surfaces such as the surface of mammalian or human skin and subsequently removable by force. Such peelable films should have an adhesive force of at least about 5 to about 25 ounces. The peelable films should not have such high adhesive force that they cause disruption to the outer layer of the skin and thereby damage the skin. Likewise, the adhesive force of the peelable films should be sufficiently high to enable the films to exfoliate and remove comedones from the skin.

These peelable films contain polyvinyl alcohols and adhesion-enhancing polymers, specifically a high molecular weight polyvinyl alcohol component having a molecular weight from about 80,000 to about 200,000 in the amount of from about 10 to about 16% by weight of the film, an adhesion enhancing polymer selected from the group consisting of polyesters, polyacrylates, polyvinylacrylates, polyurethanes, polyvinylcaprolactam and a combination thereof, said adhesion enhancing polymer component being present in the amount of from about 0.5 to about 4% by weight of the film; and a low molecular weight polyvinylalcohol component having a molecular weight from about 10,000 to about 50,000 in an amount of from about 0 to about 5% by weight of the composition. Generally, these films are less than, on average, about 100 microns in thickness, and preferably less than about 50 microns.

### Methods of Dissolving and Treating Blackheads

The invention provides a method of dissolving a blackhead by contacting the blackhead with a leave-on composition comprising a partially-neutralized fatty acid as described herein.

Contacting may be performed for at least about half an hour, or at least one (1) hour, or at least about five (5) hours, or at least about 12 hours, or at least about 18 hours. In one embodiment, contacting is performed overnight.

The invention also provides a method of treating blackheads, comprising contacting skin in need of treatment for blackheads with the leave on composition in a topical carrier as described above.

The invention also provides a method of treating blackheads, comprising contacting skin in need of treatment for blackheads with an article of manufacture as described above.

The invention also provides a cosmetic method of dissolving blackheads, comprising contacting skin in need thereof with a leave on composition in a topical carrier as described above, or comprising contacting skin in need thereof with an article of manufacture as described above.

The invention also provides a non-therapeutic method of dissolving blackheads, comprising contacting skin in need thereof with a leave on composition in a topical carrier as described above, or comprising contacting skin in need thereof with an article of manufacture as described above.

The composition or article may be applied as needed and/or as part of a regular regimen ranging from application once a week up to one or more times a day (e.g., twice a day). The amount used will vary with the age and physical condition of the end user, the duration of the treatment, the specific compound, product, or composition employed, the particular cosmetically-acceptable carrier utilized, and like factors.

The following examples further illustrate the invention.

### Example 1

A series of compounds and compositions were tested for their ability to dissolve blackheads as follows.

The blackheads were extracted from human volunteers using a comedone extracator. They were placed in a 24 or 96 well plate and submerged into approximately 100 microliters of each test material listed in Table 1 for 24 hours at room temperature (∼25° C). Gray color cards (GretagMacbeth ColorChecker™ Color Rendition Chart - Neutral 8 (0.23) CC20) were used underneath and measurements were made using a WILD M10 microscope at 40x, no agitation. The plates were covered with parafilm to avoid evaporation, and each test solution was removed at 24 hours and allowed to dry overnight. The amount of blackhead dissolved was graded by expert inspection on a scale from 1-7, 1 being not dissolved, and 7 being completely dissolved as shown in Table 1.

The results are shown in Table 2.

**TABLE 1**

| Grading Scale | |
|---|---|
| Number | Description |
| 7 | Fully Dissolved |
| 6 | Mostly dissolved, can see remnants of the original blackhead |
| 5 | More than 25% of the original blackhead seen |
| 4 | More than 50% of the original blackhead seen |
| 3 | More than 75% of the original blackhead seen |
| 2 | More than 90% of the original blackhead seen |
| 1 | More than 95% of the original blackhead seen (most similar to initial image) |

**TABLE 2**

| Results | |
|---|---|
| Test Material | Outcome at 24 hours |
| Water | 2.00±0.01 |
| Phosphate Buffered Saline | 5.25±0.96 |
| 10% Myristic acid in KOH | 6.00±0.01 |
| 10% Oleic acid in KOH | 7.00±0.01 |
| 10% Plantarein (sic - Plantaren (Lauryl Glucoside)) in water | 5.67±0.58 |
| 0.1M NaOH | 4.33±0.58 |
| Neutrogena soap (10%) diluted in water | 4.00±0.01 |
| Neutrogena Soap based cream cleanser (10%) diluted in water | 5.00±0.01 |

Further embodiments of the invention:
1. A method of dissolving a blackhead, comprising contacting the blackhead with a leave-on composition comprising a neutralized fatty acid.
2. The method of embodiment 1, wherein the composition further comprises a penetration enhancer.
3. The method of embodiment 1, wherein the penetration enhancer comprises a compound selected from the group consisting of propylene glycol, essential oils, alkyl lactates, and cetyl lactate.
4. The method of embodiment 1, wherein the composition comprises about 2.0 to about 15 % by weight of the neutralized fatty acid.
5. The method of embodiment 1, wherein the neutralized fatty acid is a saturated fatty acid selected from the group consisting of myristic acid, caprylic acid, capric acid, lauric acid, palmitic acid, stearic acid, arachidic acid, behenic acid, lignoceric acid, and cerotic acid.
6. The method of embodiment 1, wherein the neutralized fatty acid is an unsaturated fatty acid containing 14 to 22 carbons and one or more double bonds.
7. The method of embodiment 6, wherein the unsaturated fatty acid is selected from the group consisting of myristoleic acid, palmitoleic acid, sapienic acid, oleic acid, linoleic acid, alpha linoletic acid, arachidonic acid, eicosapentaenoic acid, eruicic acid, and docosahexaenoic acid.
8. The method of embodiment 1, wherein the neutralized fatty acid comprises a combination of saturated and unsaturated fatty acids.
9. The method of embodiment 1, wherein the neutralized fatty acid has been neutralized with a neutralizing agent selected from the group consisting of sodium hydroxide, potassium hydroxide, calcium hydroxide, triethanolamine, trolamine, aminomethylpropanol, and sodium bicarbonate.
10. The method of embodiment 9, wherein the mole ratio of fatty acid to neutralizing agent is about 1:0.75 to about 1:1.25.
11. The method of embodiment 1, wherein said contacting is for at least about 5 hours.
12. The method of embodiment 1, wherein said contacting is for at least about 18 hours.
13. A leave-on composition for treating blackheads, comprising a neutralized fatty acid and a topical carrier comprising a penetration enhancer.
14. The composition of embodiment 13, wherein the penetration enhancer comprises a compound selected from the group consisting of propylene glycol, essential oils, alkyl lactates and cetyl lactate.
15. The composition of embodiment 13, wherein the composition comprises about 2.0 to about 15 % by weight of the neutralized fatty acid.
16. The composition of embodiment 13, wherein the neutralized fatty acid is a saturated fatty acid selected from the group consisting of caprylic acid, capric acid, lauric acid, myristic acid, palmitic acid, stearic acid, arachidic acid, behenic acid, lignoceric acid, and cerotic acid.
17. The composition of embodiment 13, wherein the neutralized fatty acid is an unsaturated fatty acid containing 14 to 22 carbons and one or more double bonds.
18. The composition of embodiment 17, wherein unsaturated fatty acid is selected from the group consisting of myristoleic acid, palmitoleic acid, sapienic acid, oleic acid, linoleic acid, alpha linoletic acid, arachidonic acid, eicosapentaenoic acid, eruicic acid, and docosahexaenoic acid.
19. The composition of embodiment 13, wherein the neutralized fatty acid comprises a combination of saturated and unsaturated fatty acids.
20. The composition of embodiment 13, wherein the neutralized fatty acid has been neutralized with a neutralizing agent selected from the group consisting of sodium hydroxide, potassium hydroxide, calcium hydroxide, triethanolamine, trolamine, and aminomethylpropanol.
21. The composition of embodiment 20, wherein the mole ratio of fatty acid to neutralizing agent is about 1:0.75 to about 1:1.25.
22. The composition of embodiment 13, wherein the topical carrier further comprises an additional cosmetically active agent selected from the group consisting of anti-acne agents, lipid dissolvers and anti-inflammatory agents.
23. An article of manufacture comprising: 1) a leave-on composition for treating blackheads comprising a neutralized fatty acid and a topical carrier comprising a penetration enhancer; and 2) a substrate.
24. The article of embodiment 23, wherein the substrate comprises a dissolvable film.
25. The article of embodiment 23, wherein the substrate comprises a non-dissolvable film.
26. The article of embodiment 23, where the substrate comprises a non-woven material.
27. The article of embodiment 23, wherein the substrate is a mask.
28. A method of treating blackheads, comprising contacting skin in need of treatment for blackheads with a leave-on composition comprising a neutralized fatty acid and a topical carrier comprising a penetration enhancer.
29. The method of embodiment 28, wherein said contacting is for at least 5 hours.
30. The method of embodiment 28, wherein said contacting is for at least 18 hours.
31. A method of treating blackheads, comprising contacting skin in need of treatment for blackheads with an article of manufacture comprising: 1) a leave-on composition for treating blackheads comprising a neutralized fatty acid and a topical carrier comprising a penetration enhancer; and 2) a substrate.
32. The method of embodiment 31, wherein said contacting is for at least 5 hours.
33. The method of embodiment 31, wherein said contacting is for at least 18 hours.

## Claims

1. A method of dissolving a blackhead, comprising contacting the blackhead with a leave-on composition comprising a neutralized fatty acid.

2. The method of claim 1, wherein the composition further comprises a penetration enhancer, optionally wherein the penetration enhancer comprises a compound selected from the group consisting of propylene glycol, essential oils, alkyl lactates, and cetyl lactate.

3. The method of claim 1 or 2, wherein the composition comprises about 2.0 to about 15 % by weight of the neutralized fatty acid.

4. The method of any of claims 1-3, wherein:
a) the neutralized fatty acid is a saturated fatty acid selected from the group consisting of myristic acid, caprylic acid, capric acid, lauric acid, palmitic acid, stearic acid, arachidic acid, behenic acid, lignoceric acid, and cerotic acid, or
b) the neutralized fatty acid is an unsaturated fatty acid containing 14 to 22 carbons and one or more double bonds, for example an unsaturated fatty acid selected from the group consisting of myristoleic acid, palmitoleic acid, sapienic acid, oleic acid, linoleic acid, alpha linoletic acid, arachidonic acid, eicosapentaenoic acid, eruicic acid, and docosahexaenoic acid, or
c) the neutralized fatty acid comprises a combination of saturated and unsaturated fatty acids.

5. The method of any of claims 1-4, wherein the neutralized fatty acid has been neutralized with a neutralizing agent selected from the group consisting of sodium hydroxide, potassium hydroxide, calcium hydroxide, triethanolamine, trolamine, aminomethylpropanol, and sodium bicarbonate optionally, wherein the mole ratio of fatty acid to neutralizing agent is about 1:0.75 to about 1:1.25.

6. The method of any of claims 1-5, wherein said contacting is for at least about 5 hours or at least about 18 hours.

7. A leave-on composition for treating blackheads, comprising a neutralized fatty acid and a topical carrier comprising a penetration enhancer.

8. The composition of claim 7, wherein the penetration enhancer comprises a compound selected from the group consisting of propylene glycol, essential oils, alkyl lactates and cetyl lactate.

9. The composition of claim 7 or 8, wherein the composition comprises about 2.0 to about 15 % by weight of the neutralized fatty acid.

10. The composition of any of claims 7-9, wherein:
a) the neutralized fatty acid is a saturated fatty acid selected from the group consisting of caprylic acid, capric acid, lauric acid, myristic acid, palmitic acid, stearic acid, arachidic acid, behenic acid, lignoceric acid, and cerotic acid, or
b) the neutralized fatty acid is an unsaturated fatty acid containing 14 to 22 carbons and one or more double bonds, for example an unsaturated fatty acid is selected from the group consisting of myristoleic acid, palmitoleic acid, sapienic acid, oleic acid, linoleic acid, alpha linoletic acid, arachidonic acid, eicosapentaenoic acid, eruicic acid, and docosahexaenoic acid, or
c) the neutralized fatty acid comprises a combination of saturated and unsaturated fatty acids.

11. The composition of any of claims 7-10, wherein the neutralized fatty acid has been neutralized with a neutralizing agent selected from the group consisting of sodium hydroxide, potassium hydroxide, calcium hydroxide, triethanolamine, trolamine, and aminomethylpropanol, optionally wherein the mole ratio of fatty acid to neutralizing agent is about 1:0.75 to about 1:1.25.

12. The composition of any of claims 7-11, wherein the topical carrier further comprises an additional cosmetically active agent selected from the group consisting of anti-acne agents, lipid dissolvers and anti-inflammatory agents.

13. An article of manufacture comprising: 1) a leave-on composition for treating blackheads comprising a neutralized fatty acid and a topical carrier comprising a penetration enhancer; and 2) a substrate.

14. The article of claim 13, wherein the substrate comprises a dissolvable film, a non-dissolvable film, or a non-woven material, or wherein the substrate is a mask.

15. A method of treating blackheads, comprising contacting skin in need of treatment for blackheads with a leave-on composition comprising a neutralized fatty acid and a topical carrier comprising a penetration enhancer.

16. A method of treating blackheads, comprising contacting skin in need of treatment for blackheads with an article of manufacture comprising: 1) a leave-on composition for treating blackheads comprising a neutralized fatty acid and a topical carrier comprising a penetration enhancer; and 2) a substrate.

17. The method of claim 15 or 16, wherein said contacting is for at least 5 hours or at least 8 hours.
